# EUROPÄISCHE PATENTSCHRIFT

(11) **EP 3 313 342 B1**
(45) Veröffentlichungstag und Bekanntmachung des Hinweises auf die Patenterteilung: **12.02.2020**
(21) Anmeldenummer: 16731594.4
(22) Anmeldetag: 22.06.2016
(51) Int. Cl.: A61F 13/34, A61F 13/20

(54) **VERFAHREN ZUR HERSTELLUNG EINES TAMPONS**
METHOD FOR PRODUCING A TAMPON
PROCÉDÉ DE PRODUCTION D'UN TAMPON

(30) Priorität: 25.06.2015 AT 505542015
(43) Veröffentlichungstag der Anmeldung: 02.05.2018
(73) Patentinhaber: Ruggli Projects AG, 6332 Hagendorn (CH)
(72) Erfinder: LENHERR, Harald, 8222 Beringen (CH)
(74) Vertreter: IPrime Rentsch Kaelin AG
(86) Internationale Anmeldenummer: PCT/EP2016/064462
(87) Internationale Veröffentlichungsnummer: WO 2016/207242

(56) Entgegenhaltungen:
- EP-A1- 2 749 261
- WO-A1-2004/021943
- CA-A1- 2 085 923
- DE-A1- 3 634 704
- DE-A1- 19 753 665
- DE-T2- 69 311 384

## Beschreibung

Die Erfindung betrifft ein Verfahren zur Herstellung eines Tampons, welches folgende Schritte umfasst:
i) Positionieren eines Streifens aus einem Vliesstoff auf einem Streifen aus einem saugfähigen Material, sodass ein Teil des Streifens aus Vliesstoff über eine Schmalseite des Streifens aus saugfähigem Material übersteht,
ii) Verbinden des Streifens aus Vliesstoff mit dem Streifen aus saugfähigem Material,
iii) Anordnen eines Auszugsmittels an dem Streifen aus saugfähigem Material,
iv) Aufrollen des Streifens aus saugfähigem Material und des Streifens aus Vliesstoff zu einer Rolle, bei welcher der Streifen aus Vliesstoff einen Abschnitt der Mantelfläche der Rolle bedeckt,
v) Verbinden des überstehenden Teils des Streifens aus Vliesstoff mit einem mit dem Streifen aus saugfähigem Material verbundenen Abschnitt des Streifens aus Vliesstoff.

Weiters betrifft die Erfindung einen Tampon mit zumindest einem Saugkörper aus einem saugfähigen Material, wobei der Tampon ein proximales Ende und ein distales Ende und einen zwischen dem distalen Ende und dem proximalen Ende verlaufenden Mittelteil sowie ein mit dem Saugkörper verbundenes, an dem distalen Ende angeordnetes Auszugmittel aufweist.

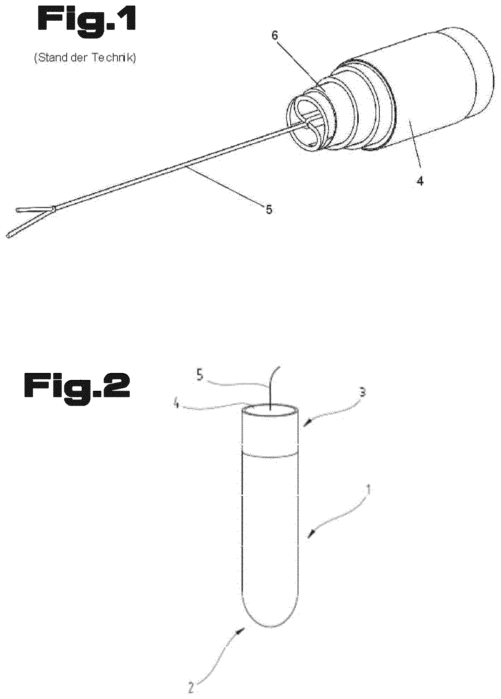

Bei bekannten Tampons die aus einem gerollten Streifen aus saugfähigem Material hergestellt sind, kann es trotz Verdichtung des Materials im Gebrauch zu einer ungewollten Loslösung von Fasern des saugfähigen Materials an einem Auszugsende kommen, vor allem beim Loslösen des Auszugmittels vom distalen Ende mit den Fingern ist dies der Fall. Da der Tampon in der Herstellung gewickelt wird, besteht bei herkömmlichen Tampons auch die Gefahr, dass ein Tampon nach dessen Gebrauch bzw. beim Entfernen (Zug am Faden) aus dem Körper "teleskopiert", wie dies in Fig. 1 veranschaulicht ist, und so allenfalls Teile des Streifens aus saugfähigem Material, beispielsweise Watte, im Körper verbleiben können.

Aus der EP1677722B1 ist ein Tampon bekannt geworden, bei welchem ein Großteil des Tampons mit einer Umhüllung aus einem wasserabweisenden bzw. wasserdichten Material versehen ist. Dies hat jedoch den Nachteil einer verringerten Flüssigkeitsaufnahme. Bei anderen Tampons tritt zusätzlich der Nachteil auf, dass sie eine geringe Durchtrittszeit für Flüssigkeit aufweisen. D.h. bis bei Tränken des Tampons mit einer Flüssigkeit ein Tropfen der Flüssigkeit an dem distalen Ende austritt. Diese Durchtrittszeit kann beispielsweise mittels einer Messung, gemäß dem EDANA Standard Test WSP 350.1.R3(12), ermittelt werden.

Es ist daher eine Aufgabe der Erfindung, die oben genannten Nachteile des Stands der Technik zu überwinden und die Durchtrittszeit für Flüssigkeit durch einen Tampon zu erhöhen.

Diese Aufgabe wird mit einem Verfahren der eingangs genannten Art erfindungsgemäß dadurch gelöst, dass in Schritt i) der Streifen aus Vliesstoff so positioniert wird, dass er auch über eine Längskante des Streifens aus saugfähigem Material übersteht, wobei ein über die Längskante des Streifens aus saugfähigem Material überstehender Abschnitt des Streifens aus Vliesstoff so groß gewählt wird, dass eine Stirnseite der Rolle von dem überstehenden Teil vollständig bedeckbar ist, und in einem Schritt iv) nachfolgenden Schritt ein distales Ende der Rolle, von welchem das Auszugsmittel absteht, mit dem überstehenden Abschnitt des Vliesstoffes mit Ausnahme einer Durchtrittsöffnung für das Auszugsmittel vollständig bedeckt und der überstehende Abschnitt des Vliesstoffes zu einer das distale Ende bis auf die Durchtrittsöffnung vollständig bedeckenden, geschlossenen Hülle verbunden wird, wobei der Vliesstoff ein Flächengewicht von mindestens 6 g/m², insbesondere zwischen 12 und 30 g/m², aufweist oder dass anstelle des Vliesstoffes eine flüssigkeitsdurchlässige Folie aus Kunststoff verwendet wird.

An dieser Stelle sei darauf hingewiesen, dass sich nach Anfertigung der Rolle und der Hülle weitere an sich bekannte Schritte, wie beispielsweise Verdichten und Pressen mittels einer Presse, zur Herstellung des fertigen Tampons anschließen können.

Die erfindungsgemäße Lösung erlaubt ein vollständiges Bedecken des Endes, an welchem das Auszugsmittel angeordnet ist, mit einem Vliesstoff. Dadurch werden ein Teleskopieren bzw. Auseinanderziehen des gewickelten Tampons und ein Faserverlust des Tampons sehr effizient unterbunden. Weiters wird an dem distalen Ende ein Auslaufschutz gegen Flüssigkeit realisiert. Durch den Auslaufschutz wird die Flüssigkeit länger in dem Tampon zurückgehalten als ohne Auslaufschutz. So lässt sich beispielsweise bei einem Tränken des Tampons mit einer Flüssigkeit die Zeit bis die Flüssigkeit an dem distalen Ende austritt gegenüber einem gleichartigen Tampon ohne Abdeckung um mindestens 3% erhöhen. Als Messmethode kommt hierbei bevorzugt der EDANA Standard Test WSP 350.1.R3(12) zum Einsatz.

Unter einem Vliesstoff, auch als Nonwoven bezeichnet, wird in dem vorliegenden Zusammenhang ein textiles Flächengebilde verstanden, welches durch Aufbringen mindestens eines Klebstoffes auf ein Fasergemisch oder auf ein Faservlies aus Synthese- und/oder Naturfasern und durch anschließendes Trocknen hergestellt wird. Kennzeichnend für einen Vliesstoff ist jedoch, dass die Fasern durch den Klebstoff in dem Vliesstoff gebunden sind. Der Vliesstoff ist für Fasern des Materials, aus welchem der Saugkörper des Tampons hergestellt ist, undurchlässig und kann darüber hinaus feuchtigkeitsdurchlässig oder aber auch wasserdicht ausgeführt sein. Der Vliesstoff kann beispielsweise auch die Form eines Netzes oder einer Lochfolie aufweisen. Auch sei an dieser Stelle darauf hingewiesen, dass der Begriff Vliesstoff als Synonym für den Begriff Nonwoven verwendet wird.

Vorteilhaft ist, wenn das saugfähige (faserförmige) Material aus einem oder mehreren Materialien wie Rayon, Baumwolle, Zellstoff, Zellstoffwatte, Tissue-Schichtstoffe, Torfmull, Bambus oder chemisch verstärkten, modifizierten oder vernetzten Zellulosefasern, gebildet ist. Dieses faserförmige Material weist eine hohe Hygroskopie auf und ermöglicht das Anhaften von hohen Flüssigkeitsmengen an den Außenseiten der Fasern und ist biologisch neutral. Es ist aber auch möglich, saugfähige (faserförmige) Materialien zu verwenden, die durch eines oder mehrere der nachstehenden, synthetischen Materialien, wie Polyesterfasern, Polyolefinfasern, saugfähige Schaumstoffe, saugfähige Schwämme, saugfähige Polymere, kapillare Kanalfasern, synthetische Fasern, überwiegend offenzelligen Polyurethan-Weichschaum oder Fasern bzw. Fäden aus Rayon oder einem Strukturtyp der kristallen Modifikation von Zellulose II gebildet ist.

Die Länge des Tampons kann beispielsweise zwischen 40 mm bis 70 mm betragen und Durchmesser größer als 11 mm sein. Der Tampon kann aber auch, insbesondere für die Tage zwischen den Menstruationsperioden, mit einem Volumen des saugfähigen Materials zur Aufnahme einer Flüssigkeitsmenge zwischen 0,5 g und 4 g bevorzugt 3,5 g, ausgebildet sein und der Saugkörper eine Länge kleiner 40 mm, bevorzugt eine Länge zwischen 40 und 10 mm oder zwischen 38 und 30 mm aufweisen.

Vorteilhaft ist es auch, wenn zumindest der Mittelteil des Saugkörpers oder ein diesen umhüllender Hüllkörper zylinderförmig ausgebildet ist, wodurch eine gleichmäßige Anlage an den Schleimhäuten der Vagina erzielt werden kann.

Es kann sich aber auch als vorteilhaft erweisen, wenn zumindest der Mittelteil des Saugkörpers oder ein diesen umhüllender Hüllkörper kegel- bzw. kegelstumpfförmig ausgebildet ist, da das Einsetzen des Alltagstampon bzw. Tampons dadurch vereinfacht werden kann.

Nach einer anderen Weiterbildung ist vorgesehen, dass sich der Kegel bzw. der Kegelstumpf vom proximalen Ende bis zum distalen Ende durchgehend erstreckt, wodurch die Entnahme des Alltagstampon bzw. Tampons vereinfacht werden kann.

Weiters ist es aber auch möglich, dass zumindest innerhalb des Mittelteils des Saugkörpers ein Kernbereich aus faserförmigem Material angeordnet ist, welcher bei gleichem Volumen zur Aufnahme einer geringeren Flüssigkeitsmenge ausgebildet ist, als das Material des Saugkörpers. Dadurch kann trotz der gewünschten und bei unterschiedlichen anatomischen Ausbildungen benötigten größeren Durchmessern des Tampons die Saugwirkung bzw. das Ausmaß der Flüssigkeitsaufnahme vor allem im Saugkörper einfacher an die benötigten Ausmaße angepasst werden.

Weiters kann aber auch, zumindest innerhalb des Mittelteils des Saugkörpers, ein Kernbereich aus einem Material angeordnet sein, welcher bei gleichem Volumen zur Aufnahme einer geringeren Flüssigkeitsmenge ausgebildet ist als das Material des Saugkörpers oder flüssigkeitsabweisend ist. Damit kann die aufzunehmende Flüssigkeitsmenge ausschließlich durch die Gestaltung und das Volumen des Saugkörpers festgelegt werden.

Der Tampon kann Markierungen an seiner Oberfläche aufweisen, er kann in verschiedenen Farben hergestellt werden. Ebenfalls kann der Tampon mit einem chemischen Indikator hergestellt werden, der die Farbe bei bestimmten Krankheiten verändert wie zum Beispiel bei Anämie, Diabetes, Hepatitis A, B oder C und HIV.

Eine zusätzliche Verbesserung kann dadurch erreicht werden, dass der Tampon und/oder der Saugkörper eine Länge von kleiner 40 mm bevorzugt eine Länge zwischen 40 und 10 mm oder zwischen 38 und 30 mm aufweist, wodurch auch die Expansion und der Druck des Tampons gegen die Gebärmutter verringert und damit eine Abdichtung und ein Abdämmen der Austrittsöffnung der Gebärmutter, die erheblichen Diskomfort erzeugen kann, verhindert wird.

Weiters ist auch eine Ausgestaltung möglich, bei der der Saugkörper über sein gesamtes Volumen aus einem saugfähigen Material gebildet ist, wodurch bei einem Tampon die Abmessungen des Durchmessers und die Menge der aufnehmbaren Flüssigkeit optimiert werden können.

Nach einer weiteren, vorteilhaften Weiterbildung ist vorgesehen, dass das saugfähige Material aus faserförmigem Material gebildet ist, wodurch die Tröpfchen der Flüssigkeit an den einzelnen Fasern anhaften können und damit eine hohe Absorptionsfähigkeit des Saugkörpers eines Alltagstampon bzw. Tampons erreicht wird.

Vorteilhaft ist aber auch, wenn das saugfähige Material aus einem komprimierten, faserförmigen Material gebildet ist, da dadurch die Handhabung des Tampons, insbesondere das Einführen und Entnehmen des Tampons, erleichtert wird.

Eine weitere vorteilhafte Weiterbildung sieht vor, dass der Saugkörper mit in Längsrichtung desselben verlaufenden Vertiefungen bzw. Rillen versehen ist und/oder dass die Vertiefungen bzw. Rillen in Richtung der Längsmittelachse des Saugkörpers wellenförmig, bevorzugt mit gleichbleibender Höhe der Amplitude verlaufen, wodurch eine Vergrößerung der Oberfläche des Tampons stattfindet. Beispielsweise ist dadurch eine vermehrte Aufnahme von Körperflüssigkeit möglich, da der Körper unterschiedliche Mengen an Körperflüssigkeit zwischen den aufeinanderfolgenden Menstruationsphasen, ausschütten kann. Ebenfalls sind diese Rillen bei der Verwendung von Einführhilfen für den Alltagstampon bzw. Tampon von Vorteil.

Eine noch höhere Oberfläche zur Feuchtigkeitsaufnahme kann dadurch erreicht werden, dass die Rillen bzw. Vertiefungen spiralförmig oder schraubenlinienförmig verlaufen.

Ein weiterer Vorteil wird dadurch erreicht, dass mehrere Rillen bzw. Vertiefungen in Umfangsrichtung über den Saugkörper verteilt angeordnet sind. Dadurch erfolgt ebenfalls eine Vergrößerung der Oberfläche des Tampons, was zur vermehrten Aufnahme von Körperflüssigkeit führt, da der Körper unterschiedliche Mengen an Körperflüssigkeit zwischen den Menstruationsphasen ausschütten kann. Für Frauen mit vermehrtem Ausfluss ist eine größere Hygiene gegeben als bei einer Slipeinlage. Ebenfalls sind diese Rillen bei der Verwendung von Alltagstampon bzw. Tampon Einführhilfen von Vorteil. Die Rillen können durch Pressen der Rolle in einer Tamponpresse hergestellt werden.

Gemäß einer vorteilhaften Ausführungsform der Erfindung kann es vorgesehen sein, dass das distale Ende der Rolle, von welchem das Auszugsmittel absteht, mit dem überstehenden Abschnitt des Vliesstoffes bei gestrecktem Auszugsmittel bedeckt wird. Durch diese Ausführungsform lässt sich gewährleisten, dass das Auszugsmittel außerhalb der Hülle aus Vliesstoff zu liegen kommt und während des Bedeckens des distalen Endes mit dem überstehenden Abschnitt die Herstellung der Hülle nicht behindert.

Eine vorteilhafte Weiterbildung der Erfindung besteht darin, dass ein umlaufender Randbereich des überstehenden Abschnitts des Streifens aus Vliesstoff zur Erzeugung der geschlossenen Hülle in Richtung des Auszugmittels gebogen wird und einander berührende Abschnitte des überstehenden Abschnittes miteinander verbunden werden.

Gemäß einer weiteren Variante der Erfindung kann es auch vorgesehen sein, dass der überstehende Abschnitt des Streifens aus Vliesstoff zur Erzeugung der geschlossenen Hülle mit einem die Mantelfläche bedeckenden Abschnitt des Streifens aus Vliesstoff verbunden wird.

Nach einer bevorzugten Ausführungsform wird der überstehende Abschnitt des Streifens aus Vliesstoff durch Verschweißen zu der Hülle verbunden. Alternativ zu einem Verschweißen können auch andere Verbindungsmethoden, wie beispielsweise Verkleben, vernähen etc., zum Einsatz kommen.

Günstigerweise kann der Streifen aus saugfähigem Material eine Länge aufweisen, deren Betrag aus einem Wertebereich stammt, dessen untere Grenze 150 mm und dessen obere Grenze 400 mm beträgt, wobei der Streifen aus Vliesstoff eine Länge aufweist, deren Betrag aus einem Wertebereich stammt, dessen untere Grenze 50 mm und dessen obere Grenze 250 mm beträgt, wobei der Streifen aus Vliesstoff in Schritt i) so positioniert wird, dass er über die Längskante des Streifens aus saugfähigem Material um eine Länge übersteht, welche im Wesentlichen einem Radius oder Durchmesser der in Schritt iv) hergestellten Rolle entspricht. Darüber hinaus kann es vorgesehen sein, dass die Breite des Streifens aus Vliesstoff größer ist, als die Breite des Streifens aus saugfähigem Material, wobei der Streifen aus Vliesstoff in Schritt i) so auf dem Streifen aus saugfähigem Material positioniert wird, dass der Streifen aus Vliesstoff mindestens drei Viertel der Breite des Streifens aus saugfähigem Material bedeckt. Bei dieser Ausführungsform der Erfindung erstreckt sich der Vliesstoff über einen großen Teil der Mantelfläche der Rolle. Auf diese Weise lässt sich gewährleisten, dass eine Reibung zwischen den Schleimhäuten der Vagina und der äußeren Oberfläche des Tampons verringert werden und ein Faserverlust auch zumindest in dem Mittelteil des Tampons effizient verhindert werden kann. Natürlich kann sich der Streifen aus Vliesstoff auch über die gesamte Breite des Streifens aus saugfähigem Material erstrecken, sodass der gesamte Tampon von dem Vliesstoff umgeben ist.

Gemäß einer vorteilhaften Variante der Erfindung kann es vorgesehen sein, dass das distale Ende des Tampons mit einer wasserdichten oder wasserabweisenden Beschichtung versehen wird.

Eine weitere vorteilhafte Ausführungsform der Erfindung besteht darin, dass das distale Ende des Tampons mit einer Abdeckung aus einem wasserdichten oder wasserabweisenden Material versehen wird.

Die oben genannte Aufgabe lässt sich auch mit einem Tampon der eingangs genannten Art erfindungsgemäß dadurch lösen, dass der Tampon eine zumindest das distale Ende mit Ausnahme einer Durchtrittsöffnung für das Auszugsmittel vollständig bedeckende Abdeckung in Form einer geschlossenen Hülle aus einem Vliesstoff mit einem Flächengewicht von mindestens 6 g/m², insbesondere zwischen 12 und 30 g/m², oder aus einer flüssigkeitsdurchlässigen Folie aufweist, durch welche ein Austritt von Fasern an dem distalen Ende verhindert ist, wobei durch die Abdeckung zusätzlich ein Auslaufschutz gegen Flüssigkeit gebildet ist.

Das distale Ende des Tampons lässt sich gegen ein ungewolltes Eindringen von Flüssigkeit schützen, indem das distale Ende des Tampons eine wasserdichte oder wasserabweisende Beschichtung aufweist.

Darüber hinaus kann das distale Ende des Tampons eine Abdeckung aus einem wasserdichten oder wasserabweisenden Material aufweisen.

Zum besseren Verständnis der Erfindung wird diese anhand der nachfolgenden Figuren näher erläutert.

Es zeigen jeweils in stark vereinfachter, schematischer Darstellung:
- Fig. 1: ein Teleskopieren eines herkömmlichen, gewickelten Tampons durch Aus-übung eines Zuges an dem Auszugsmittel;
- Fig. 2: einen erfindungsgemäßen Tampon in einer perspektivischen Ansicht;
- Fig. 3: eine perspektivische Ansicht einer Anordnung eines saugfähigen Materials und eines Vliesstoffes gemäß einem Herstellungsschritt zur Herstellung des Tampons aus Fig. 2;
- Fig. 4: eine Draufsicht auf die Anordnung aus Fig. 3;
- Fig. 5: die Anordnung aus Fig. 3 in einem aufgerollten Zustand;
- Fig. 6: die Anordnung aus Fig. 5 mit einem durch den Vliesstoff verschlossenen distalen Ende;
- Fig. 7 bis 10: Ausführungsformen eines erfindungsgemäßen Tampons mit einer Beschichtung oder einer Abdeckung des distalen Endes aus einem wasserdichten oder wasserabweisenden Material.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene, sowie dargestellte Figur bezogen und sind diese Lageangaben bei einer Lageänderung sinngemäß auf die neue Lage zu übertragen.

Die Ausführungsbeispiele zeigen mögliche Ausführungsvarianten des erfindungsgemäßen Tampons, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Gemäß Fig. 2 umfasst ein erfindungsgemäßer Tampon 1 einen Saugkörper aus einem saugfähigen Material 6, beispielsweise Watte. Der Tampon 1 weist ein proximales Ende 2 und ein distales Ende 3 und einen zwischen dem proximalen Ende 2 und dem distalen Ende 3 verlaufenden Mittelteil auf. Von Vorteil ist es wenn der Tampon 1 an dem proximalen Ende 2 rund oder abgerundet ausgebildet ist.

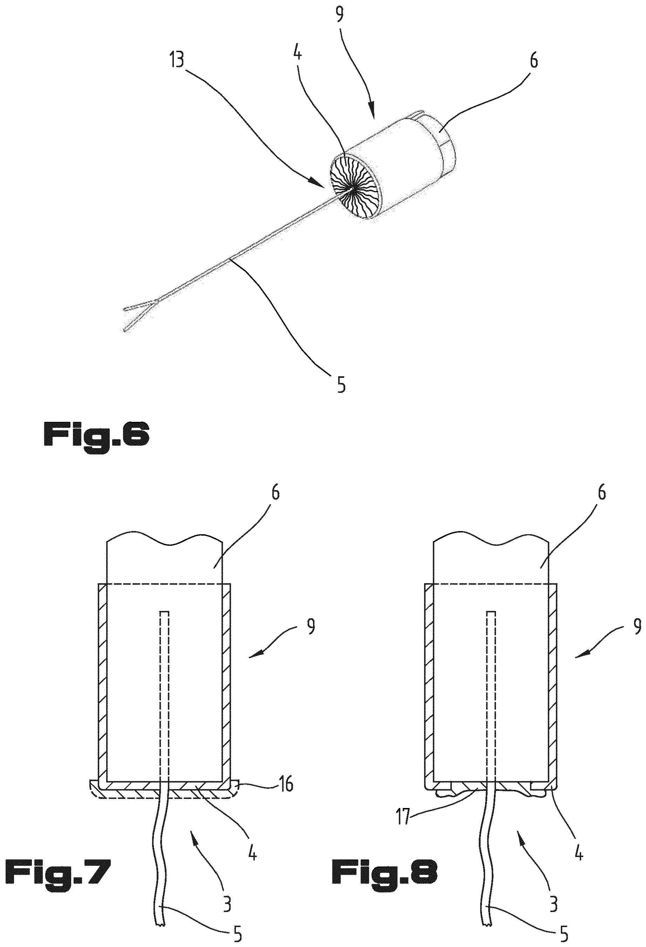

An dem distalen Ende 3 ist ein mit dem Saugkörper verbundenes Auszugmittel 5 angeordnet. An seinem distalen Ende 3 ist eine mit Ausnahme einer in Fig. 6 mit dem Bezugszeichen 13 versehenen Durchtrittsöffnung für das Auszugsmittel 5 das distale Ende 3 vollständig bedeckende Abdeckung aus einem Streifen 4 eines Vliesstoffes angeordnet. Bevorzugt weist die Durchtrittsöffnung 13 einen Durchmesser auf, welcher im Wesentlichen einem Durchmesser des Auszugsmittels 5 entspricht. Durch die Abdeckung sind ein Teleskopieren des Tampons und ein Austritt von Fasern an dem distalen Ende 3 verhindert. So ist in Fig. 1 beispielhaft dargestellt wie ein herkömmlicher Tampon durch Ausübung eines Zuges mit dem Auszugsmittel 5 auseinandergezogen (teleskopiert) werden kann. Hierbei wird eine Wicklung eines mit dem Auszugsmittel 5 verbundenen, gewickelten Streifens 6 aus saugfähigem Material in Längsrichtung auseinandergezogen, wodurch es bei herkömmlichen Tampons zu einer Auflösung des Tampons kommen kann.

Der Mittelteil des Tampons 1 kann zylinderförmig ausgebildet sein. Es ist aber auch möglich, dass zumindest der Mittelteil kegelstumpfförmig ausgebildet ist. Der Zylinder bzw. der Kegel bzw. der Kegelstumpf kann sich aber auch vom proximalen Ende bis zum distalen Ende durchgehend erstrecken. Bei all den vorgenannten Raumformen des Tampons 1 sind beliebige Querschnittsveränderungen über eine Länge des Tampons 1 möglich, wie z.B. ein wellenförmiger Verlauf oder zumindest über einen Teil des Umfanges umlaufende und / oder sich in Längsrichtung erstreckende Vertiefungen.

Eine weitere vorteilhafte Ausbildung sieht vor, dass zumindest der Mittelteil des Tampons 1 mit in Längsrichtung desselben verlaufenden Vertiefungen bzw. Rillen versehen ist und/oder dass die Vertiefungen bzw. Rillen in Richtung der Längsmittelachse des Tampons 1 wellenförmig, bevorzugt mit gleichbleibender Höhe der Amplitude verlaufen, wodurch eine Vergrösserung der Oberfläche des Tampons 1 stattfindet, wodurch eine vermehrte Aufnahme von Körperflüssigkeit möglich ist. Eine noch höhere Oberfläche zur Feuchtigkeitsaufnahme kann dadurch erreicht werden, dass die Rillen bzw. Vertiefungen spiralförmig oder schraubenlinienförmig verlaufen.

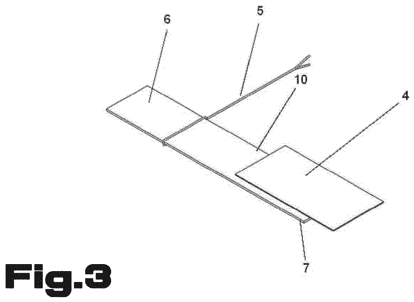

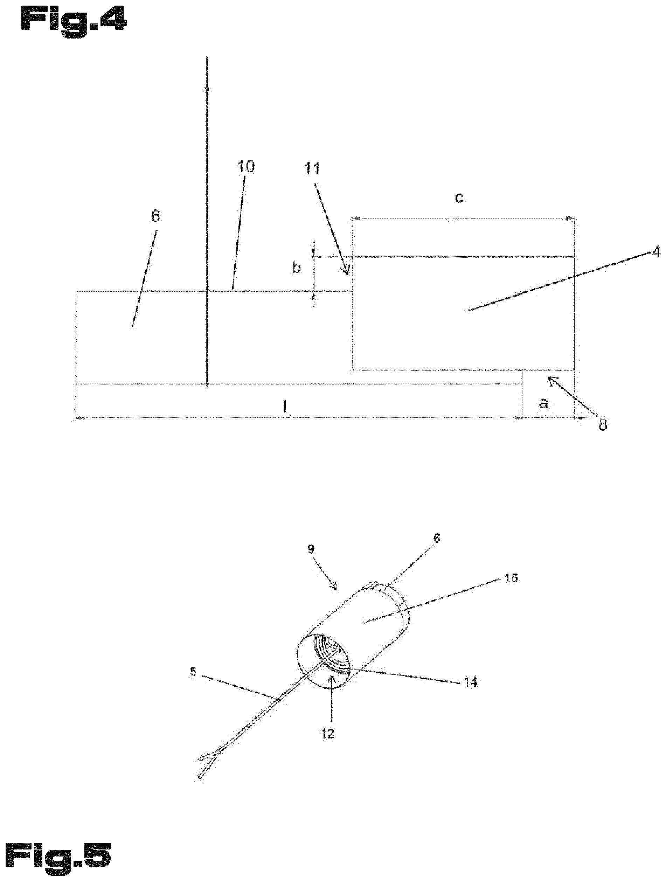

Bei einem erfindungsgemäßen Verfahren zur Herstellung des in Figur 2 dargestellten Tampons 1 wird gemäß den Figuren 3 und 4 der Streifen 4 aus einem Vliesstoff auf einem Streifen 6 aus einem saugfähigen Material positioniert, wobei ein Teil 8 des Streifen 4 über eine Schmalseite 7 des Streifens 6 aus saugfähigem Material übersteht. Weiters wird der Streifen 4 auf dem Streifen 6 so positioniert, dass er auch über eine Längskante 10 des Streifens 6 übersteht. Ein über die Längskante 10 des Streifens 6 überstehender Abschnitt 11 des Streifens 4 wird dabei so groß gewählt, dass nach einem Aufrollen der Streifen 4 und 6 zu einer Rolle 9 eine Stirnseite 12 dieser Rolle 9 mit dem überstehenden Abschnitt 11 vollständig bedeckt werden kann, wie dies in Fig. 5 und 6 dargestellt ist. Der verwendete Vliesstoff weist hierbei ein Flächengewicht von mindestens 6 g/m² insbesondere jedoch zwischen 12 und 30 g/m² auf. Anstelle des Vliesstoffes kann aber auch eine flüssigkeitsdurchlässige Folie aus Kunststoff verwendet werden. Die Folie kann beispielsweise durch Einbringen von Perforationen flüssigkeitsdurchlässig gemacht werden.

Die saugfähige Struktur des Streifens 6 lässt sich aus einer Vielzahl von Größen und Formen und aus einer Vielzahl von flüssigkeitsabsorbierenden Materialien herstellen.

Selbstverständlich ist es wünschenswert, saugfähige Materialien zu verwenden, die einen Mindestgehalt an fremdlöslichen Materialien enthalten, da das Produkt für einen bestimmten Zeitraum im Körper verbleibt. Zurückgehaltene lösliche Fremdmaterialien könnten ein Sicherheitsrisiko darstellen, wenn sie toxisch, reizend oder empfindlich sind.

Eine Liste brauchbarer Materialien enthält zellulosehaltige Materialien wie beispielsweise Rayon, Baumwolle, Zellstoff, Zellstoffwatte, Tissue-Schichtstoffe, Torfmull, Bambus und chemisch verstärkte, modifizierte oder vernetzte Zellulosefasern; synthetische Materialien wie beispielsweise Polyesterfasern, Polyolefinfasern, saugfähige Schaumstoffe, beispielsweise ein elastisch federnder Polyurethanschaumstoff, saugfähige Schwämme, äußerst saugfähige Polymere, saugfähige, gelbildende Materialien, bearbeitete Fasern wie beispielsweise kapillare Kanalfasern und Fasern mit mehreren Gliedern; synthetische Fasern oder ein äquivalentes Material oder Kombinationen von Materialien oder Mischungen daraus.

Bei Verwendung derartiger Kunststoffschäume ist es auch möglich, beispielsweise durch Temperatureinstellung bei der Herstellung der Saugkörper, partiell die Außenhaut zu verschließen, das heißt, im Außenbereich aufgrund partieller Temperaturbeeinflussung eine geschlossene Haut zu erzielen. Dadurch kann auch ein Austritt von in der Schaumstruktur aufgenommener Flüssigkeit zusätzlich erschwert oder verhindert werden. Eine derartige Ausbildung kann vor allem im proximalen Endbereich 4 des Tampons 1 von Vorteil sein.

Die Herstellung des Tampons 1 kann im Rahmen der Erfindung aus unterschiedlichsten Materialien erfolgen, die unter den Bedingungen im Bereich der Scheide bzw. Gebärmutter, das heißt bei Körpertemperaturen einen pH-Wert von ca. 4 über längere Zeit ihren Zustand aufrecht erhalten und keine Giftstoffe oder schleimhautschädlichen Lösungen oder dergleichen abgeben. Als Material für den Streifen 6 können beispielsweise Polyurethanweichschäume mit sehr niedrigem Raumgewicht und einer überwiegend offenzelligen Struktur mit Vorteilen eingesetzt werden. Durch die Verwendung von absorptionsfähigerem Material wird auch die Gefahr des Austretens von Flüssigkeit weiter reduziert. Der Vorteil für derartige, schaumförmige Strukturen liegt auch darin, dass die offenen Zellen im Auslieferungszustand oder vor dem Einführen in die Scheide mit Medikamenten bzw. Gleitmittel gefüllt werden können, die im eingesetzten Zustand an die Schleimhäute der Scheide bzw. des Uterus abgegeben werden können.

Von Vorteil ist es weiteres, wenn die für den Vliesstoff verwendeten Materialien biologisch abbaubar sind und beispielsweise aus PLA oder sonstigen, biologisch abbaubaren Kunststoffen oder Kunststoffgemischen bzw. Fäden oder Fasern bestehen oder recyclierten Kunststoffen, wie R-PP, R-PET oder dergleichen gegebenenfalls in unterschiedlichen Mischungen oder als mehrlagige Teile aus diesen Materialien gebildet sind.

Bevorzugter Weise weist der Streifen 6 eine Länge 1 auf, deren Betrag aus einem Wertebereich stammt, dessen untere Grenze 150 mm und dessen obere Grenze 400 mm beträgt. Der Streifen 4 kann eine Länge c aufweisen, deren Betrag aus einem Wertebereich stammt, dessen untere Grenze 50 mm und dessen obere Grenze 250 mm beträgt. Der Streifen 4 wird bevorzugt so positioniert wird, dass er über die Längskante 10 des Streifens 6 zumindest um eine Breite b übersteht, welche im Wesentlichen einem Radius der Rolle 9 entspricht.

Gemäß einem bevorzugten Ausführungsbeispiel weist der Streifen 6 eine Länge 1 von 255 mm auf, wobei der Teil 8 eine Länge a von 30 mm und der Streifen 4 eine Gesamtlänge c von 127 mm aufweist. Der Abschnitt 11 weist hierbei eine Breite b von 30 mm auf.

Darüber hinaus kann eine gesamte Breite des Streifens 4 größer sein als eine gesamte Breite des Streifens 6. Der Streifen 4 wird besonders bevorzugt auf dem Streifen 6 so positioniert, dass der Streifen 4 sich über mindestens drei Viertel der Breite des Streifens 6 erstreckt, wie dies in den Figuren 3 und 4 dargestellt ist.

Nach dem Positionieren des Streifens 4 auf dem Streifen 6 wird der Streifen 4 mit dem Streifen 6 verbunden. Das Verbinden der Streifen 4 und 6 kann beispielsweise durch Verschwei-βen, Verkleben, Nähen oder jede andere geeignete Verbindungsform erfolgen.

In einem weiteren Schritt wird ein Auszugsmittel 5, beispielsweise ein Faden oder eine Schnur, an dem Streifen 6 aus saugfähigem Material befestigt. Bevorzugterweise wird der Streifen 6, wie in den Figuren 2 und 3 dargestellt, mit dem Auszugsmittel 5 umschlungen. Sodann werden die Streifen 4 und 6 zu der Rolle 9 aufgewickelt. Hierbei wird die Wickelrichtung so gewählt, dass der Streifen 4 einen Abschnitt einer Mantelfläche der Rolle 9 bedeckt, wie dies beispielsweise in Fig. 5 dargestellt ist.

Der überstehende Teil 8 des Streifens 4 wird mit einem mit dem Streifen 6 verbundenen Abschnitt 15 des Streifens 4 zu einem geschlossenen Ring verbunden, beispielsweise durch Verschweißen und/ oder Verkleben und/oder vernähen.

Ein distales Ende der Rolle 9, von welchem das Auszugsmittel 5 absteht, wird mit dem überstehenden Abschnitt 11 des Streifens 4 mit Ausnahme der Durchtrittsöffnung 13 für das Auszugsmittel 5 vollständig bedeckt. Der überstehende Abschnitt 11 des Streifens 4 wird hierauf zu einer das distale Ende bis auf die Durchtrittsöffnung 13 vollständig bedeckenden, geschlossenen Hülle verbunden.

Das Bedecken des distalen Endes der Rolle 9 mit dem überstehenden Abschnitt 11 des Streifens 4 erfolgt besonders bevorzugt bei gestrecktem Auszugsmittel 5. Das Bedecken des distalen Endes der Rolle 9 kann durch Biegen bzw. Falten eines umlaufenden Randbereichs 14 des überstehenden Abschnitts 11 in Richtung des (gestreckten) Auszugmittels 5 erfolgen, wobei die Durchtrittsöffnung 13 frei gelassen wird, oder das Auszugsmittel vor dem Einklappen auf die Stirnseite der Rolle durch den überstehenden Abschnitt 11 hindurchgeführt wird. Dies kann z.B. mittels einer Nadel, an der das Auszugmittel befestigt ist, erfolgen. Einander berührende Abschnitte des überstehenden Abschnittes 11 können dann miteinander verbunden werden, beispielsweise durch Verschweißen, Vernähen oder Verkleben, sodass eine geschlossene Abdeckung entsteht. Bei dieser Ausführungsform ist es von Vorteil, wenn der Abschnitt 11 zumindest etwa einem Radius der Rolle 9 entspricht.

Alternativ oder zusätzlich zu der in dem letzten Abschnitt genannten Variante könnte der Abschnitt 11 des Streifens 4 zur Erzeugung der geschlossenen Hülle auch mit dem an der Mantelfläche der Rolle angeordneten Abschnitt 15 aus Vliesstoff verbunden werden. Ist die Breite b des Abschnittes 11 etwas größer als der Durchmesser der Rolle 9, so kann ein Bereich des Abschnittes 11 über die gesamte distale Stirnfläche 12 der Rolle 9 geklappt und mit dem Abschnitt 15 verbunden werden. Hierbei hat es sich als vorteilhaft herausgestellt, wenn der über die Stirnseite der Rolle 9 geklappte Teil des Abschnittes 11 eine Öffnung, beispielsweise in Form eines Schlitzes zur Durchführung des Auszugsmittels 5 aufweist.

Die in Figur 6 dargestellte Rolle 9 mit der Abdeckung des distalen Endes in Form einer geschlossenen Hülle aus dem Nonwoven bzw. Vliesstoff 4 stellt einen Vorformling dar, der in weiterer Folge in an sich bekannten Herstellungsschritten, wie beispielsweise Pressen und Verdichten in einer Presse, zu dem in Figur 2 dargestellten Tampon 1 weiterverarbeitet wird. Alternativ oder zusätzlich zu einer Abdeckung des distalen Endes mit Vliesstoff kann das distale Ende auch mit einer flüssigkeitsdurchlässigen Folie aus Kunststoff abgedeckt werden. Durch die Abdeckung des distalen Endes wird ein Auslaufschutz realisiert.

So weist ein erfindungsgemäßer Tampon eine deutlich höhere Durchtrittszeit für eine Flüssigkeit auf als ein herkömmlicher Tampon. In Tabelle 1 sind gemäß dem EDANA Standard Test WSP 350.1.R3(12) ermittelten Ergebnisse für Tampons mit und ohne Abdeckung am distalen Ende einander gegenübergestellt. Die Testbedingungen wurden für alle Tampons gleich gewählt, um vergleichbare Aussagen zu erhalten. Bei diesen Tests wurde die Zeit gemessen bis der erste Tropfen der Testflüssigkeit, der sogenannten Syngina Flüssigkeit, zu Boden fällt.

**Tabelle 1:**

| | Nonwoven 14g/m² | Nonwoven 12g/m² |
|---|---|---|
| Mit Abdeckung | Mittelwert: 14 min. 59 sek. | Mittelwert: 15 min. 48 sek. |
| Ohne Abdeckung | Mittelwert: 13 min. 46 sek. | Mittelwert: 15 min. 02 sek. |
| Prozentuale Verbesserung des Auslaufschutzes | 8,84% | 5,1 % |

Gemäß Fig. 7 kann der Tampon 1 an seinem distalen Ende mit einer wasserdichten oder wasserabweisenden Beschichtung 16 versehen werden. Die Beschichtung 16 kann durch Benetzen oder Tränken des distalen Endes des Tampons 1 mit einer Imprägnierungsflüssigkeit, beispielsweise einem Lack, Wachs, einem Harz, etc. erfolgen.

Die Beschichtung 16 kann wie in Fig. 7 dargestellt, das distale Ende ganz oder wie in Fig. 8 dargestellt das distale Ende teilweise bedecken. In Fig. 8 ist die Beschichtung mit dem Bezugszeichen 17 versehen. Die Beschichtung des Tampons 1 mit der wasserdichten oder wasserabweisenden Beschichtung 16, 17 kann unabhängig von den anderen Herstellungsschritten des Tampons 1 erfolgen und für sich alleine genommen Gegenstand einer Erfindung sein.

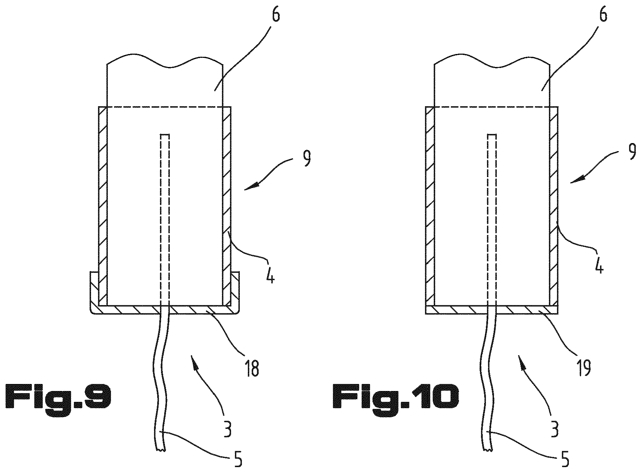

Nach Fig. 9 kann das distale Ende auch mit einer Abdeckung 18 aus einem wasserundurchlässigen oder wasserabweisenden Material gefertigt sein. Wie in Fig. 9 dargestellt, kann die Abdeckung 18 als Hülse gefertigt sein, welche auch Seitenflächen des Tampons 1 umfasst, jedoch den Großteil der Seitenflächen des Tampons nicht bedeckt.

Gemäß Fig. 10 kann die Abdeckung 19 aus wasserundurchlässigen oder wasserabweisenden Material auch nur das distale Ende bedecken ohne Seitenflächen des Tampons 1 zu umfassen. In diesem Fall ist die Abdeckung 19 als Scheibe ausgeführt.

Von Vorteil ist es, wenn die für die Abdeckungen 18, 19 verwendeten Materialien biologisch abbaubar sind und beispielsweise aus PLA oder sonstigen, biologisch abbaubaren Kunststoffen oder Kunststoffgemischen bzw. Fäden oder Fasern bestehen oder recyclierten Kunststoffen, wie R-PP, R-PET oder dergleichen gegebenenfalls in unterschiedlichen Mischungen oder als mehrlagige Teile aus diesen Materialien gebildet sein.

Das Versehen des Tampons 1 mit der wasserdichten oder wasserabweisenden Abdeckungen 18 und 19 kann ebenfalls unabhängig von den anderen Herstellungsschritten des Tampons 1 erfolgen und für sich alleine genommen Gegenstand einer Erfindung sein.

Die Abdeckungen 18, 19 kann direkt an den Streifen 4 aufgebracht sein (beispielsweise durch Verschweißen, Verkleben oder Vernähen). Der Streifen 4 kann vor oder nach dem Schritt i) aufgebracht werden.

Der Ordnung halber sei abschließend darauf hingewiesen, dass zum besseren Verständnis des Aufbaus des Tampons dieser bzw. dessen Bestandteile teilweise unmaßstäblich und/oder vergrößert und/oder verkleinert dargestellt wurden.

### Bezugszeichenaufstellung

- 1: Tampon
- 2: proximales Ende
- 3: distales Ende
- 4: Streifen aus Vliesstoff
- 5: Auszugsmittel
- 6: Streifen aus saugfähigem Material
- 7: Schmalseite
- 8: Teil des Streifens aus Vliesstoff
- 9: Rolle
- 10: Längskante
- 11: Überstehender Abschnitt
- 12: Stirnseite der Rolle
- 13: Durchtrittsöffnung
- 14: Randbereich
- 15: Abschnitt des Streifens aus Vliesstoff
- 16: Beschichtung
- 17: Beschichtung
- 18: Abdeckung
- 19: Abdeckung
- a: Länge
- b: Breite
- c: Gesamtlänge
- 1: Länge

## Patentansprüche

1. Verfahren zur Herstellung eines Tampons (1), welches folgende Schritte umfasst:
i) Positionieren eines Streifens (4) aus einem Vliesstoff auf einem Streifen (6) aus einem saugfähigen Material, sodass ein Teil (8) des Streifens (4) aus Vliesstoff über eine Schmalseite (7) des Streifens (6) aus saugfähigem Material übersteht,
ii) Verbinden des Streifens (4) aus Vliesstoff mit dem Streifen (6) aus saugfähigem Material,
iii) Anordnen eines Auszugsmittels (5) an dem Streifen (6) aus saugfähigem Material,
iv) Aufrollen des Streifens (6) aus saugfähigem Material und des Streifens (4) aus Vliesstoff zu einer Rolle (9), sodass der Vliesstoff zumindest einen Abschnitt einer Mantelfläche der Rolle (9) bedeckt,
v) Verbinden des überstehenden Teils (8) des Streifens (4) aus Vliesstoff mit einem mit dem Streifen (6) aus saugfähigem Material verbundenen Abschnitt (15) des Streifens(4) aus Vliesstoff,
**dadurch gekennzeichnet, dass**
in Schritt i) der Streifen (4) aus Vliesstoff so positioniert wird, dass er auch über eine Längskante (10) des Streifens (6) aus saugfähigen Material übersteht, wobei ein über die Längskante (10) des Streifens (6) aus saugfähigem Material überstehender Abschnitt (11) des Streifens (4) aus Vliesstoff so groß gewählt wird, dass eine Stirnseite (12) der Rolle (9) von dem überstehenden Abschnitt (11) vollständig bedeckbar ist, und in einem Schritt iv) nachfolgenden Schritt ein distales Ende der Rolle (9), von welchem das Auszugsmittel (5) absteht, mit dem überstehenden Abschnitt (11) des Streifens (4) aus Vliesstoff mit Ausnahme einer Durchtrittsöffnung (13) für das Auszugsmittel (5) vollständig bedeckt und der überstehende Abschnitt (11) des Streifens (4) aus Vliesstoff zu einer das distale Ende bis auf die Durchtrittsöffnung (13) vollständig bedeckenden, geschlossenen Hülle verbunden wird, wobei der Vliesstoff ein Flächengewicht von mindestens 6 g/m², insbesondere zwischen 12 und 30 g/m², aufweist oder dass anstelle des Vliesstoffes eine flüssigkeitsdurchlässige Folie aus Kunststoff verwendet wird.

2. Verfahren nach Anspruch 1, **dadurch gekennzeichnet, dass** das distale Ende der Rolle (9), von welchem das Auszugsmittel absteht, mit dem überstehenden Abschnitt (11) des Streifens (4) aus Vliesstoff bei gestrecktem Auszugsmittel (5) bedeckt wird.

3. Verfahren nach Anspruch 1 oder 2, **dadurch gekennzeichnet, dass** ein umlaufender Randbereich (14) des überstehenden Abschnitts (11) des Streifens (4) aus Vliesstoff zur Erzeugung der geschlossenen Hülle in Richtung des Auszugmittels (5) gebogen wird und einander berührende Abschnitte des überstehenden Abschnittes (11) miteinander verbunden werden.

4. Verfahren nach einem der Ansprüche 1 bis 3, **dadurch gekennzeichnet, dass** der überstehende Abschnitt (11) des Streifens aus Vliesstoff zur Erzeugung der geschlossenen Hülle mit einem die Mantelfläche bedeckenden Abschnitt (15) aus Vliesstoff verbunden wird.

5. Verfahren nach einem der Ansprüche 1 bis 4, **dadurch gekennzeichnet, dass** der überstehende Abschnitt (11) des Streifens (4) aus Vliesstoff durch Verschweißen zu der Hülle verbunden wird.

6. Verfahren nach einem der Ansprüche 1 bis 5, **dadurch gekennzeichnet, dass** der Streifen (6) aus saugfähigem Material eine Länge (1) aufweist, deren Betrag aus einem Wertebereich stammt, dessen untere Grenze 150 mm und dessen obere Grenze 400 mm beträgt, wobei der Streifen (4) aus Vliesstoff eine Länge (c) aufweist, deren Betrag aus einem Wertebereich stammt, dessen untere Grenze 50 mm und dessen obere Grenze 250 mm beträgt, wobei der Streifen (4) aus Vliesstoff in Schritt i) so positioniert wird, dass er über die Längskante (10) des Streifens (6) aus saugfähigem Material um eine Breite (b) übersteht, welche im Wesentlichen einem Radius der in Schritt iv) hergestellten Rolle (9) entspricht.

7. Verfahren nach einem der Ansprüche 1 bis 6, **dadurch gekennzeichnet, dass** eine Breite des Streifens (4) aus Vliesstoff größer ist als eine Breite des Streifens (6) aus saugfähigem Material, wobei der Streifen (4) aus Vliesstoff in Schritt i) so auf dem Streifen (6) aus saugfähigem Material positioniert wird, dass der Streifen (4) aus Vliesstoff mindestens drei Viertel der Breite des Streifens (6) aus saugfähigem Material bedeckt.

8. Verfahren nach einem der Ansprüche 1 bis 7, **dadurch gekennzeichnet, dass** das distale Ende des Tampons (1) mit einer wasserdichten oder wasserabweisenden Beschichtung (16, 19) versehen wird.

9. Verfahren nach einem der Ansprüche 1 bis 8, **dadurch gekennzeichnet, dass** das distale Ende des Tampons (1) mit einer Abdeckung (17, 18) aus einem wasserdichten oder wasserabweisenden Material versehen wird.

10. Tampon (1) mit zumindest einem Saugkörper aus einem saugfähigen Material (6), wobei der Tampon (1) ein proximales Ende (2) und ein distales Ende (3) und einen zwischen dem distalen Ende und dem proximalen Ende verlaufenden Mittelteil sowie ein mit dem Saugkörper verbundenes, an dem distalen Ende (3) angeordnetes Auszugmittel (5) aufweist, **dadurch gekennzeichnet, dass** der Tampon eine zumindest eine das distale Ende (3) mit Ausnahme einer Durchtrittsöffnung (13) für das Auszugsmittel (5) vollständig bedeckende Abdeckung in Form einer geschlossenen Hülle aus einem Streifen (4) aus Vliesstoff mit einem Flächengewicht von mindestens 6 g/m², insbesondere zwischen 12 und 30 g/m², oder aus einer flüssigkeitsdurchlässigen Folie aufweist, durch welche ein Austritt von Fasern an dem distalen Ende (3) verhindert ist, wobei durch die Abdeckung zusätzlich ein Auslaufschutz gegen Flüssigkeit gebildet ist.

11. Tampon nach Anspruch 10, **dadurch gekennzeichnet, dass** das distale Ende des Tampons (1) eine wasserdichte oder wasserabweisende Beschichtung aufweist.

12. Tampon nach Anspruch 10 oder 11, **dadurch gekennzeichnet, dass** das distale Ende des Tampons (1) eine Abdeckung aus einem wasserdichten oder wasserabweisenden Material aufweist.

## Claims

1. Method for producing a tampon (1), comprising the following steps:
i) positioning a strip (4) made of a nonwoven material on a strip (6) made of an absorbent material, such that a part (8) of the strip (4) made of nonwoven material projects over a narrow side (7) of the strip (6) made of absorbent material,
ii) bonding the strip (4) made of nonwoven material to the strip (6) made of absorbent material,
iii) arranging a pull-out means (5) on the strip (6) made of absorbent material,
iv) rolling up the strip (6) made of absorbent material and the strip (4) made of nonwoven material to form a roll (9), such that the nonwoven material covers at least a portion of a lateral surface of the roll (9),
v) bonding the projecting part (8) of the strip (4) made of nonwoven material to a portion (15) of the strip (4) made of nonwoven material that is bonded to the strip (6) made of absorbent material,
**characterized in that**,
in step i), the strip (4) made of nonwoven material is positioned in such a way that it also projects over a longitudinal edge (10) of the strip (6) made of absorbent material, wherein a portion (11) of the strip (4) made of nonwoven material that projects over the longitudinal edge (10) of the strip (6) made of absorbent material is selected to be of a size such that an end face (12) of the roll (9) can be completely covered by the projecting portion (11), and, in a step that follows step iv), a distal end of the roll (9), from which end the pull-out means (5) protrudes, is completely covered with the projecting portion (11) of the strip (4) made of nonwoven material except for a through-opening (13) for the pull-out means (5), and the projecting portion (11) of the strip (4) made of nonwoven material is bonded to form a closed sheath which completely covers the distal end up to the through-opening (13), wherein the nonwoven material has a weight per unit area of at least 6 g/m², in particular between 12 and 30 g/m², or **in that**, instead of the nonwoven material, a liquid-permeable film of plastic is used.

2. Method according to Claim 1, **characterized in that** the distal end of the roll (9), from which the pull-out means protrudes, is covered with the projecting portion (11) of the strip (4) made of nonwoven material when the pull-out means (5) is extended.

3. Method according to Claim 1 or 2, **characterized in that** an encircling peripheral region (14) of the projecting portion (11) of the strip (4) made of nonwoven material is bent in the direction of the pull-out means (5) to create the closed sheath, and mutually contacting portions of the projecting portion (11) are bonded to one another.

4. Method according to one of Claims 1 to 3, **characterized in that** the projecting portion (11) of the strip made of nonwoven material is, to create the closed sheath, bonded to a portion (15) made of nonwoven material that covers the lateral surface.

5. Method according to one of Claims 1 to 4, **characterized in that** the projecting portion (11) of the strip (4) made of nonwoven material is bonded to form the sheath by heat-sealing.

6. Method according to one of Claims 1 to 5, **characterized in that** the strip (6) made of absorbent material has a length (1), the magnitude of which comes from a range of values with a lower limit of 150 mm and an upper limit of 400 mm, wherein the strip (4) made of nonwoven material has a length (c), the magnitude of which comes from a range of values with a lower limit of 50 mm and an upper limit of 250 mm, wherein, in step i), the strip (4) made of nonwoven material is positioned in such a way that it projects over the longitudinal edge (10) of the strip (6) made of absorbent material by a width (b) which corresponds substantially to a radius of the roll (9) produced in step iv).

7. Method according to one of Claims 1 to 6, **characterized in that** a width of the strip (4) made of nonwoven material is larger than a width of the strip (6) made of absorbent material, wherein, in step i), the strip (4) made of nonwoven material is positioned on the strip (6) made of absorbent material in such a way that the strip (4) made of nonwoven material covers at least three quarters of the width of the strip (6) made of absorbent material.

8. Method according to one of Claims 1 to 7, **characterized in that** the distal end of the tampon (1) is provided with a water-impermeable or water-repellent coating (16, 19).

9. Method according to one of Claims 1 to 8, **characterized in that** the distal end of the tampon (1) is provided with a covering (17, 18) made of a water-impermeable or water-repellent material.

10. Tampon (1) with at least one absorbent element made of an absorbent material (6), wherein the tampon (1) has a proximal end (2) and a distal end (3) and a middle part running between the distal end and the proximal end, and has a pull-out means (5) bonded to the absorbent element and arranged at the distal end (3), **characterized in that** the tampon has an at least one covering which completely covers the distal end (3) except for a through-opening (13) for the pull-out means (5) and is in the form of a closed sheath consisting of a strip (4) made of nonwoven material with a weight per unit area of at least 6 g/m², in particular between 12 and 30 g/m², or consisting of a liquid-permeable film, by means of which an emergence of fibres at the distal end (3) is prevented, wherein the covering additionally forms a means of protection against the leakage of liquid.

11. Tampon according to Claim 10, **characterized in that** the distal end of the tampon (1) has a water-impermeable or water-repellent coating.

12. Tampon according to Claim 10 or 11, **characterized in that** the distal end of the tampon (1) has a covering made of a water-impermeable or water-repellent material.

## Revendications

1. Procédé de fabrication d'un tampon (1), qui comprend les étapes suivantes :
i) le positionnement d'une bande (4) en un non-tissé sur une bande (6) en un matériau absorbant, de telle sorte qu'une partie (8) de la bande (4) en un non-tissé dépasse au-dessus d'un côté étroit (7) de la bande (6) en un matériau absorbant,
ii) la liaison de la bande (4) en un non-tissé avec la bande (6) en un matériau absorbant,
iii) l'agencement d'un moyen d'extraction (5) sur la bande (6) en un matériau absorbant,
iv) l'enroulement de la bande (6) en un matériau absorbant et de la bande (4) en un non-tissé en un rouleau (9), de telle sorte que le non-tissé recouvre au moins une section d'une surface d'enveloppe du rouleau (9),
v) la liaison de la partie dépassante (8) de la bande (4) en un non-tissé avec une section (15) de la bande (4) en un non-tissé reliée avec la bande (6) en un matériau absorbant,
**caractérisé en ce que**
lors de l'étape i), la bande (4) en un non-tissé est positionnée de telle sorte qu'elle dépasse également au-dessus d'un bord longitudinal (10) de la bande (6) en un matériau absorbant, une section (11) de la bande (4) en un non-tissé dépassant au-dessus du bord longitudinal (10) de la bande (6) en un matériau absorbant étant choisie d'une taille telle qu'un côté avant (12) du rouleau (9) puisse être entièrement recouvert par la section dépassante (11), et, lors d'une étape suivant l'étape iv), une extrémité distale du rouleau (9), de laquelle le moyen d'extraction (5) dépasse, est entièrement recouverte avec la section dépassante (11) de la bande (4) en un non-tissé à l'exception d'une ouverture de passage (13) pour le moyen d'extraction (5), et la section dépassante (11) de la bande (4) en un non-tissé est reliée en une gaine fermée qui recouvre entièrement l'extrémité distale à l'exception de l'ouverture de passage (13), le non-tissé présentant un poids surfacique d'au moins 6 g/m², notamment compris entre 12 et 30 g/m², ou **en ce qu'**à la place du non-tissé, un film perméable aux liquides en matière plastique est utilisé.

2. Procédé selon la revendication 1, **caractérisé en ce que** l'extrémité distale du rouleau (9), de laquelle le moyen d'extraction dépasse, est recouverte avec la section dépassante (11) de la bande (4) en un non-tissé lorsque le moyen d'extraction (5) est étiré.

3. Procédé selon la revendication 1 ou 2, **caractérisé en ce qu'**une zone de bord périphérique (14) de la section dépassante (11) de la bande (4) en un non-tissé est pliée dans la direction du moyen d'extraction (5) pour former la gaine fermée, et des sections en contact les unes avec les autres de la section dépassante (11) sont reliées les unes avec les autres.

4. Procédé selon l'une quelconque des revendications 1 à 3, **caractérisé en ce que** la section dépassante (11) de la bande en un non-tissé est reliée avec une section (15) en un non-tissé recouvrant la surface d'enveloppe pour former la gaine fermée.

5. Procédé selon l'une quelconque des revendications 1 à 4, **caractérisé en ce que** la section dépassante (11) de la bande (4) en un non-tissé est reliée pour former la gaine par soudure.

6. Procédé selon l'une quelconque des revendications 1 à 5, **caractérisé en ce que** la bande (6) en un matériau absorbant présente une longueur (l), dont le montant est dans une plage de valeurs dont la limite inférieure est de 150 mm et dont la limite supérieure est de 400 mm, la bande (4) en un non-tissé présentant une longueur (c) dont le montant est dans une plage de valeurs dont la limite inférieure est de 50 mm et dont la limite supérieure est de 250 mm, la bande (4) en un non-tissé étant positionnée à l'étape i) de telle sorte qu'elle dépasse au-dessus du bord longitudinal (10) de la bande (6) en un matériau absorbant d'une largeur (b), qui correspond essentiellement à un rayon du rouleau (9) fabriqué à l'étape iv).

7. Procédé selon l'une quelconque des revendications 1 à 6, **caractérisé en ce qu'**une largeur de la bande (4) en un non-tissé est supérieure à une largeur de la bande (6) en un matériau absorbant, la bande (4) en un non-tissé étant positionnée à l'étape i) sur la bande (6) en un matériau absorbant de telle sorte que la bande (4) en un non-tissé recouvre au moins les trois quarts de la largeur de la bande (6) en un matériau absorbant.

8. Procédé selon l'une quelconque des revendications 1 à 7, **caractérisé en ce que** l'extrémité distale du tampon (1) est munie d'un revêtement étanche à l'eau ou repoussant l'eau (16, 19).

9. Procédé selon l'une quelconque des revendications 1 à 8, **caractérisé en ce que** l'extrémité distale du tampon (1) est munie d'un recouvrement (17, 18) en un matériau étanche à l'eau ou repoussant l'eau.

10. Tampon (1) comprenant au moins un corps absorbant en un matériau absorbant (6), le tampon (1) comprenant une extrémité proximale (2) et une extrémité distale (3) et une partie centrale disposée entre l'extrémité distale et l'extrémité proximale, ainsi qu'un moyen d'extraction (5) relié avec le corps absorbant, agencé à l'extrémité distale (3), **caractérisé en ce que** le tampon comprend un au moins un recouvrement qui recouvre entièrement l'extrémité distale (3) à l'exception d'une ouverture de passage (13) pour le moyen d'extraction (5), sous la forme d'une gaine fermée en une bande (4) en un non-tissé ayant un poids surfacique d'au moins 6 g/m², notamment compris entre 12 et 30 g/m², ou en un film perméable aux liquides, qui empêche une sortie de fibres à partir de l'extrémité distale (3), le recouvrement formant en outre une protection contre les fuites de liquides.

11. Tampon selon la revendication 10, **caractérisé en ce que** l'extrémité distale du tampon (1) comprend un revêtement étanche à l'eau ou repoussant l'eau.

12. Tampon selon la revendication 10 ou 11, **caractérisé en ce que** l'extrémité distale du tampon (1) comprend un recouvrement en un matériau étanche à l'eau ou repoussant l'eau.
